# EUROPÄISCHE PATENTSCHRIFT

(11) **EP 2 714 105 B1**
(45) Veröffentlichungstag und Bekanntmachung des Hinweises auf die Patenterteilung: **01.07.2015**
(21) Anmeldenummer: 12726066.9
(22) Anmeldetag: 29.05.2012
(51) Int. Cl.: A61L 15/24, A61L 15/28

(54) **FUNKTIONALISIERTE WUNDAUFLAGE**
FUNCTIONALIZED WOUND DRESSING
PANSEMENT FONCTIONNALISÉ

(30) Priorität: 26.05.2011 EP 11167735
(43) Veröffentlichungstag der Anmeldung: 09.04.2014
(73) Patentinhaber: BioCELL Gesellschaft für Biotechnologie mbH, 51766 Engelskirchen (DE)
(72) Erfinder: VOGLER, Elisabeth, 53809 Ruppichteroth (DE); GLOCK, Wolfram, 65187 Wiesbaden (DE)
(74) Vertreter: Von Kreisler Selting Werner - Partnerschaft von Patentanwälten und Rechtsanwälten mbB
(86) Internationale Anmeldenummer: PCT/EP2012/060003
(87) Internationale Veröffentlichungsnummer: WO 2012/160217

(56) Entgegenhaltungen:
- EP-A1- 1 435 247
- WO-A1-93/19709
- WO-A2-2007/087888
- WO-A2-2008/000720
- US-A1- 2002 128 579
- US-A1- 2011 027 344
- "[TEXTUS] WUNDTHERAPIE MIT SYSTEM, Aquafaser-Verbände", online, 24. Juli 2010 (2010-07-24), XP002680076, Gefunden im Internet: URL:http://www.tricks-zur-wundversorgung.d e/resources/100430-3+Aquafaser.pdf [gefunden am 2012-07-17]
- Voggenreiter, Dold: "Wundtherapie", 2009, Georg Thieme Verlag, XP002680133, ISBN: 9783131361424 Seiten 28-30, das ganze Dokument

## Beschreibung

Gegenstand der vorliegenden Erfindung sind funktionalisierte Wundauflagen und ihre Verwendung.

Im Stand der Technik sind eine Vielzahl von Wundauflagen unterschiedlichster Aufbauten bekannt, die primär dazu dienen, das Eindringen von Fremdkörpern in eine Wunde zu verhindern und Blut oder Wundsekret aufzusaugen.

WO 2008/000720 A2 betrifft eine Wundauflage mit einer Absorberschicht aus ersten und zweiten Filamenten, wobei die ersten Filamente Polyethylenterephtalate mit einem Mantel aus Polyolefinen sind und in den Mantel eine Silberquelle eingebunden ist und die zweiten Filamente Absorber Filamente aus einem Kern aus Polyacrylnitril und einem Mantel aus Polyacryl sind.

Aufgabe der vorliegenden Erfindung war es, Wundauflagen bereitzustellen, um weitergehende Funktionen in die Wundauflage einzubringen. Überraschenderweise kann die Aufgabe gelöst werden durch eine Wundauflage mit einer Absorberschicht aus einem Funktionsvlies umfassend mindestens erste und zweite Filamente,
wobei die ersten Filamente einen Kern aus Polyacrylnitril und einen Mantel aus Polyacryl umfassen,
wobei die zweiten Filamente Alginatfasern sind, wobei die Filamente der Absorberschicht zusammen eine Helix bilden.

Bevorzugt ist die Wundauflage in der Lage, 15 bis 40 g Wasser pro g Wundauflage zu absorbieren.

Überraschenderweise sind die ersten Filamente - neben ihrer Fähigkeit zur Feuchtigkeitsabsorbtion - auch in der Lage, unerwünschte Substanzen wie beispielsweise Proteasen oder freie Sauerstoff-Radikale zu binden und hierdurch die Wundheilung zu fördern.

In der modernen Wundversorgung zeigte sich kürzlich (The journal of clinical investigation Vol 121, 3, March 2011), dass Eisenionen in ihrer Wirkung auf Macrophagen eine äußerst wichtige Rolle in der Kette zur Wundheilung und im Beitrag zur Chronifizierung und Entchronifizierung zeigen. Eisenüberladung der Macrophagen verhindert das Umschalten zum gesunden Heilungsverlauf.

Überraschenderweise sind die ersten Filamente bestehend aus Polyacrylnitril und Polyacryl neben ihrer Saugfähigkeit auch in der Lage, verstärkt Eisenionen aus der Wundumgebung aufzunehmen und zu komplexieren und damit bioanorganisch positiv in Richtung Entchronifizierung zu wirken.

Fasern natürlichen Ursprungs, d.h. Fasern aus natürlichen Polymeren, also Alginatfasern werden als zweites Filament verwendet.

Diese zeichnen sich durch eine hohe Hautverträglichkeit, gute Abbaubarkeit, preiswerte Herstellungskosten sowie eine hohe Biokompatibilität aus.

Die zweiten Filamente sind Alginatfasern. Alginatfasern sind zur Verwendung in Wundauflagen, z.B. Alginatkompressen, bereits bekannt. Sie haben absorbierende Eigenschaften und dienen der Feuchtigkeitsstabilisierung. Kommen Calciumalginate in Kontakt mit Natriumsalzen, quellen die Fasern und verwanden sich in ein Gel, das feuchtigkeitsspendend oder -regulierend wirkt. Die Herstellung von Alginatfasern ist beispielsweise beschrieben in US 2011/0027344. Entsprechende Fasern sind beispielsweise von der Smartfiber AG unter der Marke "Smartcel/SeaCell" kommerziell erhältlich.

Überraschenderweise zeigt die erfindungsgemäße Kombination von Filamenten aus Polyacrylnitril/Polyacryl mit Alginatfasern neben wesentlicher Erhöhung der Feuchteaufnahme überraschenderweise eine deutlich verringerte Tendenz an der Wunde zu haften, was den traumatischen Effekt beim Verbandwechsel reduziert. Alginate reduzieren außerdem auf Grund ihrer guten Verträglichkeit toxische Effekte.

Eine geeignete Garnstärke liegt im Bereich von 2,3 bis 2,9 dtex.

Cellulosefasern haben absorbierende Eigenschaften. Sie zeigen eine hohe Aufnahmegeschwindigkeit für Flüssigkeiten und eine gute Aufnahmekapazität. Sie dienen der Verbesserung der Feuchtigkeitsstabilität durch gelierende Effekte. Sie sind weiterhin in der Lage, Eiweißspaltprodukte zu binden. Sie sind gesundheitlich unbedenklich und als Lebensmittelzusatzstoffe zugelassen.

Cellulosehohlfasern können mit verschiedenen Inhaltsstoffen gefüllt werden. Während des Einsatzes degradiert die Cellulosehülle und setzt den Inhalt frei. Solche Fasern sind aus DE 30 44 435 C2 bekannt. Sie sind kommerziell erhältlich von der Firma Cordia Dow und z.B. vom Thüringischen Institut für Textil- und Kunststoffforschung unter der Bezeichnung SpV 574. Das Gewicht beträgt dabei z.B. ca. 300 dtex.

Carboxymethylcellulosefasern sind beispielsweise unter der Marke OASIS von der Firma Akzo Nobel oder von der Firma Acordis erhältlich.

In der modernen feuchten Wundversorgung ist bekannt, dass sowohl ein feuchtes wie auch ein warmes Klima für die Wundheilung und Beschleunigung der Wundheilung von Bedeutung sind. Die Kombination mit Celluloseacetat zeigt überraschenderweise einen stark wärmenden Effekt, der über dem von bisherig bekannten Kombinationen liegt und vor allem bei Wunden mit einem erhöhten Wärmebedarf z.B. bei schlecht durchbluteter Umgebung (chronisch venöse Insuffizienz) einen positiven Einfluss zeigt.

Carbonfasern dienen insbesondere der Geruchsneutralisierung von bakteriellen Abbauprodukten. Sie sind kommerziell erhältlich unter der Marke SPC711 von der Firma Carbon-Werke. Geeignete Garnstärken betragen 500 bis 900 tex.

Carbonfasern eignen sich besonders für große Wundauflagen, da sie eine hohe Reißfestigkeit beisteuern. Dies ermöglicht Wundauflagen mit einer Größe von über 10 cm x 30 cm, sogar bis 60 oder 90 cm Länge so zu verstärken, dass beim Entfernen der angefeuchteten und/oder feucht gewordenen Auflage kaum bis keine Rückstände auf der Wunde verbleiben. Die Auflage lässt sich im Gegensatz zu anderen Wundauflagen in einem Stück ohne Rückstände auf der Wunde entfernen, was die Eingriffs- und Behandlungsdauer beim Entfernen für den Patienten wesentlich verkürzt.

Ein Verstärken mit Carbonfaser löst außerdem das Problem, dass die Dicke der Auflage bei größerem Format nicht zunimmt, der Tragekomfort also verbessert wird.

Chitosanfasern wirken antimikrobiell, fungizid und viruzid. Solche Fasern sind beispielsweise von der Firma Medovent unter der Bezeichnung "Chitosan filament" erhältlich. Typische Durchmesser liegen bei 0,05 bis 0,5 mm. Überraschenderweise zeigt die Kombination aus Polyacrylnitril/Polyacrylfasern in Kombination mit Chitosanfasern nicht nur stark blutstillende, sondern auch juckreizlindernde Eigenschaften.

In einer Ausführungsform ist die Absorberschicht mit einer weiteren Schicht laminiert. "Laminiert" umfasst alle Verfahren zum Verbinden von zwei Schichten, insbesondere Kleben und Verschweißen von Schichten, wobei die Verbindung voll- oder teilflächig erfolgen kann.

Zur Laminierung kann beispielsweise eine wundnahe Schicht aus einem hydrophoben Material eingesetzt werden. Hierfür eignen sich beispielsweise Polyolefine wie Polyethylen. Es kann aber auch mit einem silikonbasierten Material laminiert werden. Solche Materialien sind beispielsweise aus US 5,635,201 bekannt.

Weitere denkbare Schichten für eine Laminierung sind kohlebeschichtete Netze. Solche Netze sind beispielsweise in US 2006/0211972 beschrieben.

Es können auch weitere Schichten vorhanden sein, die sich auf der wundabgewandten Seite befinden.

In einer Ausführungsform ist das Funktionsvlies wundabseitig mit einer das Vlies überlappenden oder nicht überlappenden Folie versehen, wobei bevorzugt eine PU-Folie verwendet wird. Diese Folie kann dazu dienen die Abdampfungsrate zu reduzieren und damit ein feuchtes Wundmilieu zu erhalten und das Funktionsvlies zu fixieren. Wundauflagen mit wundabseitigen PU-Folien sind im Markt bekannt und werden beispielsweise von der Firma Smith & Nephew unter der Marke Allevyn adherent vertrieben.

In einigen Ausführungsformen der Erfindung umfasst das Funktionsvlies zusätzlich eine Beladung mit mindestens einer Substanz, z.B. mit einem Wirkstoff. Das Beladen kann z.B. durch Tränken der Wundauflage oder von Schichten der Wundauflage erfolgen. Andere Verfahren sind Imprägnieren, Aufsprühen etc. Geeignete Stoffe für die Beladung sind insbesondere:
- Polyhexanid, wie dies unter der Marke Prontosan von der Firma B Braun erhältlich ist.
- Medical Honey, wie er von der Firma Advancis Medical als Activon honey erhältlich ist.
- Madensekret, wie es von der Firma Biomonde in Form von Fliegenmaden (bspw. Lucilia sericata) erhältlich ist.
- Hyaluron, wie es von der Firma Biocell unter der Marke [TEXTUS]heal erhältlich ist.
- Wirkstoffe, die naszierenden Sauerstoff freisetzen, wie sie unter der Marke Biosept von der Firma GlucoMetrix AG erhältlich sind.
- Hydrogel, wie es von der Firma Johnson & Johnson unter der Marke NU-GEL erhältlich ist.

Eine weitere Ausführungsform der Erfindung betrifft die Einbindung eines Stücks Kohlenstoff in die Vliesstruktur. Solche Materialien sind beispielsweise in US 2002/0128579 beschrieben.

Das erfindungsgemäße Funktionsvlies enthält mindestens zwei Filamente, es können aber auch drei, vier oder mehr Filamente enthalten sein. Ein Beispiel für ein drittes Filament sind Filamente, die einen Kern aus einem Polyethylenterephtalat und einen Mantel aus Polyolefin umfassen und im Mantel eine Silberquelle eingebunden haben.

Die Filamente der Absorberschicht bilden zusammen Helices.

Es können als dritte oder vierte Filamente auch weitere Filamente enthalten sein, beispielsweise eine Kombination von Alginatfasern und Cellulosefasern. Soweit die Materialien silberdotiert sind, eignet sich neben Fasern von Trevira, in die Silberionen eingeschmolzen werden, insbesondere ein Nanosilbermaterial, was unter der Bezeichnung Hygentic 9000 vertrieben wird.

Als Gewicht der Absorberschicht haben sich Gewichte von 120 bis 300, bevorzugt 120 bis 250 oder 130 bis 270 oder 130 bis 210 g/m² als besonders geeignet erwiesen.

Als Gewicht der ersten Filamente haben sich Gewichte von 20 bis 170, bevorzugt 50 bis 150 g/m² als besonders geeignet erwiesen.

Bevorzugt handelt es sich bei den Filamenten um Stapelfasern mit einer Länge von bis zu 100 mm, bevorzugt 38 bis 51 mm, bevorzugt unter 45 mm.

Die erfindungsgemäßen Wundauflagen eigenen sich insbesondere zur Behandlung von Druckgeschwüren (Dekubitus), arteriellem Ulcus, venösen Ulcus, neuropatischem Ulcus, Verbrennungen, insbesondere Verbrennungen vom Grad IIa/IIb, Post OP Wunden, radiologischen Wunden (Strahlenulcera), bakterienverursachten Wunden oder chemisch verursachten Wunden.

### Beispiel 1

### (Vergleichsbeispiel, nicht unter den Umfang der Erfindung fallend) Kombination von Polyacrylnitril/Polyacrylfilamenten mit Alginatfasern

Eine Wundauflage aus Polyacrylnitril/Polyacrylfilamenten und einem zweiten Filament Alginatfasern konnten nach Auflegen auf stark exsudierende Wunden nach 48 Stunden ohne Verkleben oder Aufreißen der Wunde abgenommen werden, was normalerweise ohne z.B. Distanznetz nicht möglich ist.

## Patentansprüche

1. Wundauflage mit einer Absorberschicht aus einem Funktionsvlies umfassend mindestens erste und zweite Filamente,
wobei die ersten Filamente einen Kern aus Polyacrylnitril und einen Mantel aus Polyacryl umfassen,
wobei die zweiten Filamente Alginatfasern sind, wobei die Filamente der Absorberschicht zusammen eine Helix bilden.

2. Wundauflage nach Anspruch 1, wobei dritte Filamente vorhanden sind, die einen Kern aus einem Polyethylenterephtalat und einen Mantel aus Polyolefin umfassen und im Mantel eine Silberquelle eingebunden ist.

3. Wundauflage nach Anspruch 1 oder 2, **dadurch gekennzeichnet, dass** die Wundauflage mindestens zwei Schichten umfasst, wobei eine wundnahe Schicht aus einem hydrophoben Material mit einer lichten Öffnungsweite von 10 bis 25 µm umfasst ist.

4. Wundauflage nach Anspruch 3, **dadurch gekennzeichnet, dass** die Wundnaheschicht aus einem Polyolefin, insbesondere Polyethylen besteht.

5. Wundauflage nach mindestens einem der Ansprüche 1 bis 4, **dadurch gekennzeichnet, dass** die Filamente der Absorberschicht unabhängig voneinander einen Durchmesser von 5 bis 40 µm oder 30 bis 500, bevorzugt 40 bis 500 µm aufweisen.

6. Wundauflage nach mindestens einem der Ansprüche 1 bis 5, **dadurch gekennzeichnet, dass** mindestens 50% der Absorptionskapazität in 30 Sek. aufgenommen werden können.

7. Wundauflage nach mindestens einem der Ansprüche 1 bis 6, **dadurch gekennzeichnet, dass** die Fähigkeit besteht, mindestens 50% der Absorptionskapazität unter 20 mm Hg Kompressionsdruck zurückzuhalten.

8. Wundauflage nach mindestens einem der Ansprüche 1 bis 7, **dadurch gekennzeichnet, dass** die Alginatfasern silberhaltig sind.

9. Wundauflage nach mindestens einem der Ansprüche 1 bis 8, **dadurch gekennzeichnet, dass** die Wundauflage in der Lage ist, 10 bis 40 g Wasser pro g Wundauflage zu absorbieren.

10. Wundauflage nach mindestens einem der Ansprüche 1 bis 9, **dadurch gekennzeichnet, dass** mindestens drei verschiedene Filamente enthalten sind.

11. Wundauflage nach mindestens einem der Ansprüche 1 bis 10, **dadurch gekennzeichnet, dass** die ersten Filamente einen Gewichtsanteil von 20 bis 170 g/m² ausmachen.

12. Wundauflage nach einem der Ansprüche 1 bis 11, **dadurch gekennzeichnet, dass** das Funktionsvlies mit einer weiteren Schicht laminiert ist, insbesondere einer Silikonschicht oder einer kohlenstoffbeschichteten Schicht.

13. Wundauflage nach einem der Ansprüche 1 bis 12, **dadurch gekennzeichnet, dass** das Funktionsvlies mit einer Substanz, insbesondere ausgewählt aus Polyhexanid, Medical Honey, Madensekret, Hyaluron, Hydrogel oder Substanzen, die naszierenden Sauerstoff freisetzen, beladen ist.

14. Wundauflage nach einem der Ansprüche 1 bis 13, **dadurch gekennzeichnet, dass** ein oder mehrere Kohlenstoffstücke in die Vliesstruktur eingebunden sind.

15. Wundauflage nach einer der Ansprüche 1 bis 14 zur Behandlung von Druckgeschwüren (Dekubitus), arteriellem Ulcus, venösen Ulcus, neuropatischem Ulcus, Verbrennungen, insbesondere Verbrennungen vom Grad IIa/IIb, Post OP Wunden, radiologischen Wunden (Strahlenulcera), bakterienverursachten Wunde oder chemisch verursachten Wunden.

## Claims

1. A wound cover comprising an absorber layer made of a functional non-woven comprising at least first and second filaments;
wherein said first filaments comprise a core of polyacrylonitrile and a sheath of polyacryl;
wherein said second filaments are alginate fibers, wherein the filaments of the absorber layer together form a helix.

2. The wound cover according to claim 1, wherein third filaments are present that comprise a core of a polyethylene terephthalate and a sheath of polyolefin, and a silver source is incorporated in the sheath.

3. The wound cover according to claim 1 or 2, **characterized in that** said wound cover comprises at least two layers including a layer proximal to the wound consisting of a hydrophobic material having an inside opening width of from 10 to 25 µm.

4. The wound cover according to claim 3, **characterized in that** said layer proximal to the wound consists of a polyolefin, especially polyethylene.

5. The wound cover according to at least one of claims 1 to 4, **characterized in that** the filaments of the absorber layer independently have a diameter of from 5 to 40 µm, or from 30 to 500 µm, preferably from 40 to 500 µm.

6. The wound cover according to at least one of claims 1 to 5, **characterized in that** at least 50% of the absorption capacity can be taken up within 30 seconds.

7. The wound cover according to at least one of claims 1 to 6, **characterized in that** it is capable of retaining at least 50% of the absorption capacity under a compressing pressure of 20 mm Hg.

8. The wound cover according to at least one of claims 1 to 7, **characterized in that** said alginate fibers contain silver.

9. The wound cover according to at least one of claims 1 to 8, **characterized in that** said wound cover is capable of absorbing from 10 to 40 g of water per g of wound cover.

10. The wound cover according to at least one of claims 1 to 9, **characterized in that** at least three different filaments are contained therein.

11. The wound cover according to at least one of claims 1 to 10, **characterized in that** said first filaments comprise a weight proportion of from 20 to 170 g/m².

12. The wound cover according to any of claims 1 to 11, **characterized in that** said functional non-woven is laminated with another layer, especially a silicone layer or a carbon-coated layer.

13. The wound cover according to any of claims 1 to 12, **characterized in that** said functional non-woven is loaded with a substance, especially one selected from polyhexanide, medical honey, maggot secretion, hyalurone, hydrogel or substances releasing nascent oxygen.

14. The wound cover according to any of claims 1 to 13, **characterized in that** one or more carbon pieces are incorporated in said non-woven structure.

15. The wound cover according to any of claims 1 to 14 for the treatment of decubitus, arterial ulcer, venous ulcer, neuropathic ulcer, burns, especially burns of degree IIa/IIb, postsurgical wounds, radiological wounds (radiation ulcers), bacterially caused wounds or chemically caused wounds.

## Revendications

1. Pansement pour plaie pourvu d'une couche d'absorption constituée d'un tissu fonctionnel non-tissé, comprenant au moins des premiers et deuxièmes filaments,
les premiers filaments comprenant un coeur en polyacrylonitrile et une gaine en polyacrylique,
les deuxièmes filaments étant des fibres d'alginate, les filaments de la couche absorbante formant conjointement une hélice.

2. Pansement selon la revendication 1, dans lequel il est prévu des troisièmes filaments qui comprennent un coeur en téréphtalate de polyéthylène et une gaine en polyoléfine, et une source d'argent est incorporé dans la gaine.

3. Pansement selon la revendication 1 ou 2, **caractérisé en ce que** le pansement pour plaie comprend au moins deux couches, une couche près de la plaie étant entourée d'une matière hydrophobe ayant une largeur d'ouverture intérieure de 10 à 25 µm.

4. Pansement selon la revendication 3, **caractérisé en ce que** la couche près de la plaie est constituée d'une polyoléfine, en particulier d'un polyéthylène.

5. Pansement selon au moins l'une des revendications 1 à 4, **caractérisé en ce que** les filaments de la couche absorbante ont, indépendamment l'un de l'autre, un diamètre de 5 à 40 µm ou de 30 à 500, de préférence de 40 à 500 µm.

6. Pansement selon au moins l'une des revendications 1 à 5, **caractérisé en ce qu'**au moins 50% de la capacité d'absorption peuvent être atteintes en 30 s.

7. Pansement selon au moins l'une des revendications 1 à 6, **caractérisé en ce que** la capacité consiste à maintenir au moins 50% de la capacité d'absorption à une pression de compression 20 mm Hg.

8. Pansement selon l'une quelconque des revendications 1 à 7, **caractérisé en ce que** les fibres d'alginate contiennent de l'argent.

9. Pansement selon l'une quelconque des revendications 1 à 8, **caractérisé en ce que** le pansement est capable d'absorber de 10 à 40 g d'eau par gramme de pansement.

10. Pansement selon au moins une des revendications 1 à 9, **caractérisé en ce qu'**au moins trois filaments différents sont inclus.

11. Pansement selon au moins une des revendications 1 à 10, **caractérisé en ce que** les premiers filaments ont un grammage de 20 à 170 g/m².

12. Pansement selon l'une quelconque des revendications 1 à 11, **caractérisé en ce que** le tissu fonctionnel non-tissé est stratifiée avec une autre couche, en particulier une couche de silicone ou une couche revêtue de carbone.

13. Pansement selon l'une des revendications 1 à 12, **caractérisé en ce que** le tissu fonctionnel non-tissé est chargé d'une substance, choisie en particulier parmi le polyhexanide, le miel médical, les sécrétions de larves de mouche, l'acide hyaluronique, un hydrogel ou des substances libérant de l'oxygène naissant.

14. Pansement selon l'une des revendications 1 à 13, **caractérisé en ce qu'**une ou plusieurs pièces de carbone sont incorporés dans la structure du tissu non-tissé.

15. Pansement selon l'une des revendications 1 à 14 servant au traitement de plaies dues à la pression (escarres), des ulcères artérielles, des ulcères veineux, des ulcères neuropathiques, des brûlures, notamment des brûlures de degré IIa/IIb, des plaies postopératoires, des plaies radiologiques (ulcères dus à des rayonnements), des plaies dues à des bactéries ou des plaies induites chimiquement.
